# EUROPEAN PATENT SPECIFICATION

(11) **EP 3 968 222 B1**
(45) Date of publication and mention of the grant of the patent: **17.01.2024**
(21) Application number: 20802264.0
(22) Date of filing: 16.04.2020
(51) Int. Cl.: G06N 3/08

(54) **CLASSIFICATION TASK MODEL TRAINING METHOD, APPARATUS AND DEVICE AND STORAGE MEDIUM**
VERFAHREN, VORRICHTUNG UND GERÄT ZUM TRAINIEREN EINES KLASSIFIZIERUNGSAUFGABENMODELLS UND SPEICHERMEDIUM
PROCÉDÉ, APPAREIL ET DISPOSITIF DE FORMATION D'UN MODÈLE DE TÂCHE DE CLASSIFICATION ET SUPPORT D'INFORMATIONS

(30) Priority: 07.05.2019 CN 201910377510
(43) Date of publication of application: 16.03.2022
(73) Proprietor: Tencent Technology (Shenzhen) Company Limited, Shenzhen, Guangdong, 518057 (CN)
(72) Inventor: SHEN, Rongbo, Shenzhen, Guangdong 518057 (CN); ZHOU, Ke, Shenzhen, Guangdong 518057 (CN); TIAN, Kuan, Shenzhen, Guangdong 518057 (CN); YAN, Kezhou, Shenzhen, Guangdong 518057 (CN); JIANG, Cheng, Shenzhen, Guangdong 518057 (CN)
(74) Representative: EP&C
(86) International application number: PCT/CN2020/085006
(87) International publication number: WO 2020/224403

(56) References cited:
- WO-A1-2017/215284
- WO-A1-2019/075771
- CN-A- 108 537 743
- CN-A- 108 763 874
- CN-A- 109 165 666
- CN-A- 109 522 973
- CN-A- 110 097 130
- HAIBO HE ET AL: "Learning from Imbalanced Data", IEEE TRANSACTIONS ON KNOWLEDGE AND DATA ENGINEERING, IEEE SERVICE CENTRE , LOS ALAMITOS , CA, US, vol. 21, no. 9, 1 September 2009 (2009-09-01), pages 1263-1284, XP011263451, ISSN: 1041-4347

## Description

This application claims priority to Chinese Patent Application No. 201910377510.3, entitled "CLASSIFICATION TASK MODEL TRAINING METHOD, APPARATUS AND, DEVICE, AND STORAGE MEDIUM" filed with the National Intellectual Property Administration, PRC, on May 7, 2019.

### FIELD

Embodiments of this application relate to the field of machine learning technologies, and in particular, to training of a classification task model.

### BACKGROUND

The machine learning technology realizes a good performance in processing classification tasks. For example, a classification task model is constructed based on deep neural networks, and the model is trained based on appropriate training samples. The trained classification task model may be used to process classification tasks such as classification tasks of image recognition and speech recognition.

In a training dataset used for training a classification task model, the numbers of training samples included in different classes may be imbalanced. For example, the number of positive samples is far less than the number of negative samples. Such a training dataset may be referred to as a class imbalanced dataset. If a classification task model is trained by using a class imbalanced dataset, the finally trained classification task model may have a poor performance.
The patent application No. CN109522973A discloses a medical big data classification method and system based on a generative adversarial network and semi-supervised learning, and the system comprises a data collection module which is used for collecting medical big data, and obtaining a large amount of medical data and medical images with high data dimension and high class mark uncertainty; The data processing module is used for preprocessing the acquired medical data and medical images; The algorithm application module is used for initializing and training the sub-learners, marking the unlabeled medical data and the unlabeled medical images, and expanding the labeled medical data and the labeled medical images; And the auxiliary decision module is used for classifying the medical big data of the test set. The data processing module further comprises a medical data dimension reduction module, an image processing module, a data classification module and a medical data processing module; The algorithm application module further comprises a training sample generation module, a training module, a marking module, an expansion module and an integration module. And the accuracy of medical big data classification is improved.

### SUMMARY

An artificial intelligence-based method and apparatus for training a classification task model, a device, and a storage medium are provided according to embodiments of this application, which can be used to solve the technical problem that a high accurate classification task model cannot be obtained by using an up-sampling method in the related art. The technical solutions are described as follows.In an aspect, a method for training a classification task model based on medical images is provided in the embodiments of this application. The method is performed by a computer device, and the method includes:
training an initial feature extractor by using a first dataset to obtain a feature extractor, the first dataset being a class imbalanced dataset which includes first class samples and second class samples, the first class samples being negative samples, and the second class samples being positive samples, the number of the first class samples being greater than the number of the second class samples, the first dataset being determined based on medical images, the first dataset comprising a plurality of sub-images of non-lesion regions and lesion regions that are extracted from the medical images;
constructing a generative adversarial network, the generative adversarial network including the feature extractor and an initial feature generator, the initial feature generator being configured to generate a feature vector of the same dimension as the feature extractor;
training the generative adversarial network by using the second class samples to obtain a feature generator, the feature generator being used to augment the second class samples in a feature space during a training process;
constructing a classification task model, the classification task model including the feature generator and the feature extractor; and
training the classification task model by using the first dataset, the trained classification task model being used to perform lesion classification based on a medical image.

In another aspect, an apparatus for training a classification task model based on medical images is provided in the embodiments of this application. The apparatus includes: a first training module, a first construction module, a second training module, a second construction module and a third training module. The first training module is configured to train an initial feature extractor by using a first dataset to obtain a feature extractor, the first dataset is a class imbalanced dataset including first class samples and second class samples, the first class samples being negative samples, and the second class samples being positive samples, and the number of the first class samples is greater than the number of the second class samples, the first dataset comprises a plurality of sub-images of non-lesion regions and lesion regions that are extracted from the medical images. The first dataset is determined based on medical images. The first construction module is configured to construct a generative adversarial network. The generative adversarial network includes the feature extractor and an initial feature generator, and the initial feature generator is configured to generate a feature vector of the same dimension as the feature extractor. The second training module is configured to train the generative adversarial network by using the second class samples to obtain a feature generator, the feature generator is used to augment the second class samples in a feature space during a training process. The second construction module is configured to construct a classification task model, and the classification task model includes the feature generator and the feature extractor. The third training module is configured to train the classification task model by using the first dataset. The trained classification task model is used to perform lesion classification based on a medical image.

In another aspect, a computer device is provided in the embodiments of this application, which includes: a processor, a communication interface, a memory, and a communication bus. The communication bus is configured to perform communication between the processor, the communication interface, and the memory. The communication interface is an interface of a communication module. The memory is configured to store a program code and transmit the program code to the processor. The processor is configured to call instructions of the program code in the memory to perform the method for training a classification task model in the foregoing aspect.

In another aspect, a storage medium is provided in the embodiments of this application, which is configured to store a computer program. The computer program is configured to perform the method for training a classification task model in the foregoing aspect.

In another aspect, a computer program product including instructions is provided in the embodiments of this application. The instructions, when run on a computer, cause the computer to perform the method for training a classification task model in the foregoing aspect.

The technical solutions provided in the embodiments of this application achieve at least the following beneficial effects. According to the technical solution provided in the embodiments of this application, a feature generator is obtained by training a generative adversarial network, and minority class samples (that is, a class of training samples of the smaller number in a class imbalanced dataset) are augmented in a feature space by using the feature generator. Augmentation is performed at a feature aspect instead of simply duplicating minority class samples by up-sampling, so that overfitting in a finally trained classification task model is prevented, thus improving the accuracy of the final trained classification task model.

### BRIEF DESCRIPTION OF THE DRAWINGS

To describe the technical solutions of the embodiments of this application more clearly, the following briefly introduces the drawings required for describing the embodiments. Apparently, the drawings in the following description show only some embodiments of this application, and a person of ordinary skill in the art may still derive other drawings from these drawings without creative efforts.
FIG. 1 is a flowchart of a method for training a classification task model according to an embodiment of this application.
FIG. 2 exemplarily shows a schematic structural diagram of an initial classification task model.
FIG. 3 exemplarily shows a schematic structural diagram of a generative adversarial network.
FIG. 4 exemplarily shows a schematic structural diagram of a classification task model.
FIG. 5 exemplarily shows a diagram of an overall architecture of a technical solution of this application.
FIG. 6 and FIG. 7 exemplarily show schematic diagrams of two groups of experimental results.
FIG. 8 is a block diagram of an apparatus for training a classification task model according to an embodiment of this application.
FIG. 9 is a schematic structural diagram of a computer device according to an embodiment of this application.

### DESCRIPTION OF EMBODIMENTS

To make the objectives, technical solutions, and advantages of this application clearer, the following further describes embodiments of this application in detail with reference to the drawings.

According to the technical solution provided in the embodiments of this application, a feature generator is obtained by training a generative adversarial network, and minority class samples (that is, a class of training samples of the smaller number in a class imbalanced dataset) are augmented in a feature space by using the feature generator. Augmentation is performed at a feature aspect instead of simply duplicating minority class samples by up-sampling, so that overfitting in a finally trained classification task model is prevented, thus improving the accuracy of the final trained classification task model.

In the embodiments of this application, machine learning (ML) in an artificial intelligence (AI) technologies and deep learning (DL) in the machine learning are involved, which includes various types of artificial neural networks (ANNs).

AI represents a theory, method, technology and application system which use digital computers or machines controlled by digital computers to simulate, extend and expand human intelligence, perceive the environment, acquire knowledge, and use knowledge to obtain optimum results. In other words, AI represents a comprehensive technology of computer sciences. AI intends to understand the essence of intelligence and produce a new kind of intelligent machine that can react in a similar way to human intelligence. AI intends to study the design principles and implementation methods of various intelligent machines, to enable the machines to have the functions of perception, reasoning and decision-making.

AI technology is a comprehensive discipline, and involves a wide range of fields, including both hardware-level technology and software-level technology. Basic AI technologies generally include technologies such as a sensor, a dedicated AI chip, cloud computing, distributed storage, a big data processing technology, an operating/interaction system, and electromechanical integration. AI software technologies mainly include several major directions such as a computer vision technology, a speech processing technology, a natural language processing technology, and machine learning/deep learning.

In the embodiments of this application, a computer device that performs the method for training a classification task model may have machine learning capabilities, so as to train the classification task model by means of the machine learning capabilities.

Machine learning is an interdisciplinarity, and involves multiple disciplines such as a probability theory, statistics, an approximation theory, a convex analysis, and an algorithm complexity theory. The machine learning specializes in studying how a computer simulates or implements a human learning behavior to obtain new knowledge or skills, and reorganize an existing knowledge structure, so as to keep improving its performance. The machine learning is the core of artificial intelligence, is a basic way to make the computer intelligent, and is applied to various fields of artificial intelligence. Machine learning and deep learning usually include technologies such as an artificial neural network.

The classification task model in the embodiments of this application is a machine learning model obtained by training through machine learning and is configured to process classification tasks. The classification task model may be a deep learning classification task model, that is, a classification task model constructed based on a deep neural network, for example, a classification task model constructed based on a deep convolutional neural network. In addition to performing a classification task of lesion recognition based on medical images, the classification task model may be configured to perform classification tasks such as image recognition and speech recognition. The specific application scenarios of the classification task model are not limited in the embodiments of this application.

In the method provided in the embodiments of this application, the steps may be performed by a computer device. The computer device is an electronic device such as a personal computer (PC) or a server with data calculation, processing, and storage capabilities.

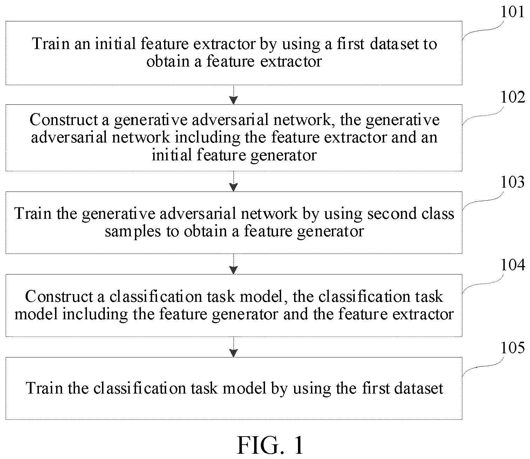

Reference is made to FIG. 1, which is a flowchart of a method for training a classification task model according to an embodiment of this application. The method may include the following steps 101 to 105.

In step 101, an initial feature extractor is trained by using a first dataset to obtain a feature extractor. The first dataset is a class imbalanced dataset including first class samples and second class samples, and the number of the first class samples is greater than the number of the second class samples.

The first class samples and the second class samples are two different classes of samples in the first dataset. The first class samples are negative samples, and the second class samples are positive samples. The number of the first class samples is greater than the number of the second class samples. That is, the first class samples may be referred to as majority class samples, and the second class samples may be referred to as minority class samples. In most scenarios, the number of the negative samples is larger or even much larger than the number of the positive samples. Therefore, the first class samples may be negative samples, and correspondingly, the second class samples are positive samples.

The feature extractor is a part configured to extract sample features in the classification task model, and the feature extractor is also referred to as an encoder. The classification task model includes a feature extractor and a classifier. An output of the feature extractor is connected to an input of the classifier. The feature extractor is configured to extract a feature vector from an inputted sample of the model. The classifier is configured to determine a class of the inputted sample based on the feature vector. For example, in a case that the classification task model is used for image recognition, the feature extractor is configured to perform mapping encoding on an inputted image, and output a feature vector with a dimension smaller than that of pixels of the inputted image, so that the feature extractor obtains a non-linear, local-to-global feature mapping, which combines low-level visual features and high-level semantic information.

In an exemplary embodiment, the classification task model is constructed based on a deep convolutional neural network, and the feature extractor may include multiple convolutional layers. For example, the classification task model is an Inception-v3 model. The Inception-v3 model is a deep neural network model, which may achieve a good performance on image classification tasks. In addition, another advantage of the Inception-v3 model is that a pre-trained Inception-v3 model may be used as an initialized classification task model without having to initialize parameters of a classification task model randomly, which facilitates improving the training efficiency of a model. The classifier may be a normalized exponential function (Softmax) classifier or another classifier, which is not limited in the embodiments of this application.

In an exemplary embodiment, step 101 includes the following sub-steps 1 and 2.

In sub-step 1, an initial classification task model is constructed. The initial classification task model includes the initial feature extractor and an initial classifier.

As described above, the initial classification task model may be a pre-trained Inception-v3 model.

In sub-step 2, the initial classification task model is trained by using the first dataset to obtain the feature extractor. The feature extractor is obtained by initially training the initial feature extractor.

The first dataset includes first class samples and second class samples, and each training sample is provided with a label corresponding to its class. For example, a label of a first class sample is 1, and a label of a second class sample is 0. Alternatively, a label of the first class sample is 0, and a label of the second class sample is 1. Training samples (including the first class samples and the second class samples) in the first dataset are inputted into the initial classification task model, a classification result outputted by the model is compared with the labels, and a loss function value corresponding to the model is calculated. A gradient of each parameter in the model is calculated based on the loss function value by using a backpropagation algorithm. Finally, the parameter in the model is updated based on the gradient, and a frequency of the update is controlled by a learning rate. The loss function may be a cross entropy (CE) loss function.

If the initial classification task model meets a training stop condition, the training of the model is stopped, and an initially trained classification task model is obtained. The initially trained classification task model includes an initially trained feature extractor. The initially trained feature extractor is used in a generative adversarial network as described below. The training stop condition of the initial classification task model may be set in advance. For example, the training stop condition may be that the accuracy of the model reaches a preset requirement, the number of times for training reaches a preset number, or a time length of training reaches a preset time length, which is not limited in the embodiments of this application.

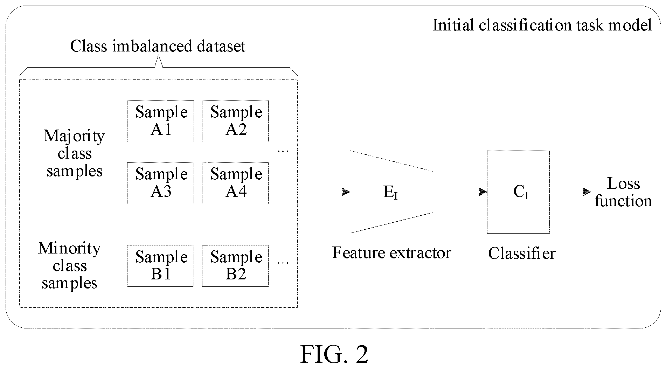

FIG. 2 exemplarily shows a schematic structural diagram of an initial classification task model. The initial classification task model includes a feature extractor E_{I} and a classifier C_{I}. An input of the feature extractor E_{I} serves as an input of the model. An output of the feature extractor E_{I} is connected to an input of the classifier C_{I}. An output of the classifier Ci serves as an output of the model. The initial classification task model is trained by using the first dataset (including majority class samples and minority class samples) to obtain an initially trained classification task model. The initially trained classification task model includes an initially trained feature extractor E_{I} and an initially trained classifier C_{I}.

In step 102, a generative adversarial network is constructed. The generative adversarial network includes the feature extractor and an initial feature generator.

In the generative adversarial network, an output of the feature generator and an output of the feature extractor are connected to an input of the domain classifier.

The feature extractor is obtained by initially training the initial feature extractor in step 101.

The initial feature generator is configured to generate a feature vector of the same dimension as the feature extractor. For example, if a dimension of a feature vector outputted by the feature extractor is 20, a dimension of a feature vector generated by the initial feature generator is also 20. The initial feature generator may be alternatively constructed by using multiple convolutional layers. If the initial feature generator includes six convolutional layers, a size of a convolution kernel of the first five convolutional layers is represented as 3*3, and a size of a convolution kernel of the last convolutional layer is represented as 1*1. The numbers of outputted feature maps of the convolutional layers are respectively 64, 128, 256, 512, 1024, and 2048. Each convolutional layer may be followed by a batch norm layer and an activation function layer, such as a rectified linear unit (ReLU) layer.

In a possible implementation, the generative adversarial network may further include a domain classifier. The domain classifier is configured to distinguish between a feature vector outputted by the feature extractor and a feature vector outputted by the feature generator. The domain classifier adjusts the feature generator through adversarial learning, so that a feature vector outputted by the domain classifier is as close as possible to the feature vector outputted by the feature extractor. Through such an adversarial learning process, model parameters of max-min game equilibrium are determined.

In step 103, the generative adversarial network is trained by using the second class samples to obtain a feature generator.

During the process of training the generative adversarial network, parameters of the feature extractor are fixed, that is, the parameters of the feature extractor are not updated. An input of the feature extractor includes the second class samples, that is, minority class samples, and an output of the feature extractor includes a feature vector extracted from the second class samples.

Input of the initial feature generator includes a superposition of a priori data and noisy data, and output of the initial feature generator includes a feature vector of the same dimension as the feature extractor. The a priori data may be extracted from the second class samples of the first dataset, or may be extracted from samples in a second dataset that are of the same class as the second class samples. The second dataset may be another dataset different from the first dataset in the same task. The noisy data may be random noisy data. Taking priori data being a 64*64 image as an example, the noisy data may be a 64*64 image, while pixel values of pixels in the image of the noisy data are randomly generated. The priori data is superposed to the noisy data, that is, a weighted sum is performed on pixel values of pixels at the same position in the a priori data and in the noisy data, to finally obtain a superimposed image.

The initial feature generator extracts the feature vector from the superimposed image. In addition, in consideration of that the number of network layers of the feature generator may be small, the input of the feature generator cannot be excessively large. Therefore, the a priori data may be a small-size sample image obtained by scaling down a sample image, for example, a 64*64 sample image.

In the embodiments of this application, in a possible implementation, the input of the initial feature generator does not completely include the noisy data. If the feature vector similar to a real sample is generated completely based on the noisy data, there is a lack of effective constraint. Therefore, the input of the initial feature generator is a superposition of the priori data and the noisy data, which can prevent problems of non-convergence and potential failure in the training process of the generative adversarial network, thereby increasing the robustness of the generative adversarial network.

In an exemplary embodiment, step 103 includes the following sub-steps 1 to 6.

In sub-step 1, a first parameter update and a second parameter update are performed during each training process of the generative adversarial network. The first parameter update includes: assigning a first label to an input of the feature extractor, and assigning a second label to an input of the feature generator.

In sub-step 2, a first loss function value of the domain classifier is calculated.

In sub-step 3, parameters of the domain classifier are updated based on the first loss function value.

In sub-step 4, the second parameter update includes: blocking the input of the feature extractor, and assigning the first label to the input of the feature generator.

In sub-step 5, a second loss function value of the domain classifier is calculated.

In sub-step 6, parameters of the feature generator are updated based on the second loss function value.

During a training process of the generative adversarial network, the initial feature generator and the domain classifier are adversarial to each other. That is, two backpropagation calculations are performed during each training process. In the first backpropagation calculation, parameters of the initial feature generator are fixed, and parameters of the domain classifier are updated. In the second backpropagation calculation, the parameters of the domain classifier are fixed, and the parameters of the initial feature generator are updated. The first label and the second label are two different labels. For example, the first label is 1, and the second label is 0. Alternatively, the first label is 0 and the second label is 1.

For example, a label of 1 is assigned to the input of the feature extractor, and a label of 0 is assigned to the input of the initial feature generator. A first loss function value of the domain classifier is calculated, and the parameters of the domain classifier are adjusted by backpropagation based on the first loss function value. Next, the input of the feature extractor is blocked, the label of 1 is assigned to the input of the initial feature generator, a second loss function value of the domain classifier is calculated, and the parameters of the initial feature generator are adjusted by backpropagation based on the second loss function value.

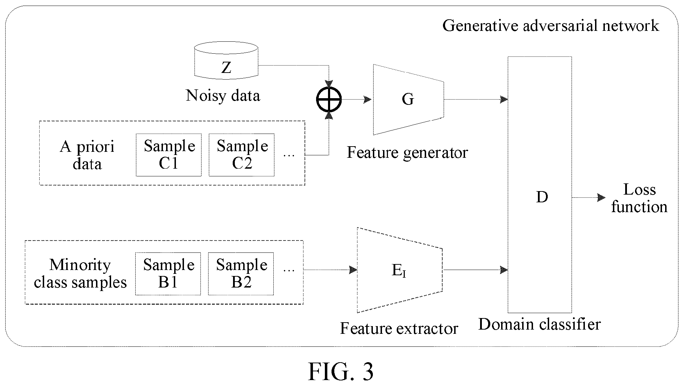

As shown in FIG. 3, which exemplarily shows a schematic structural diagram of a generative adversarial network. The generative adversarial network includes a feature extractor E_{I}, a feature generator G, and a domain classifier D. An output of the feature extractor E_{I} and an output of the feature generator G are connected to an input of the domain classifier D. An input of the feature generator G includes a superposition of priori data and noisy data, and an input of the feature extractor E_{I} includes minority class samples in a first dataset. The feature generator G is used in the following classification task model.

In step 104, a classification task model is constructed. The classification task model includes the feature generator and the feature extractor. The classification task model may further include a classifier.

In the classification task model, an output of the feature generator and an output of the feature extractor are connected to an input of the classifier.

The feature generator is obtained by training the generative adversarial network in step 103. The feature extractor and the classifier in this step adopt the same structure and configuration as that in the initial classification task model in step 101. In an embodiment, the feature extractor in this step is initialized with the parameters of the feature extractor trained in step 101.

In Step 105, the classification task model is trained by using the first dataset. The feature generator is configured to augment the second class samples in a feature space.

During a training process of the classification task model, for an original class imbalanced first dataset, the feature generator obtained by training the generative adversarial network is used to augment minority class samples in the feature space, so that a class imbalanced learning task is transformed to a class balanced learning task, and a re-trained classification task model is obtained.

In an exemplary embodiment, the classification task model further includes a data cleaning unit. Abnormal feature vectors outputted by the feature generator and the feature extractor are filtered out by using the data cleaning unit. The data cleaning unit may be a functional unit realized by software, hardware, or a combination of software and hardware. Abnormal feature vectors generated by the feature generator are suppressed by using appropriate data cleaning technology (such as a Tomek Link algorithm), so that the accuracy of the final trained classification task model is further improved.

In an exemplary embodiment, by using the data cleaning unit, a feature vector pair that meets a preset condition may be selected from the feature vectors outputted by the feature generator and the feature extractor. The feature vector pair that meets the preset condition includes two feature vectors with different labels and a similarity that conforms to a threshold, for example, a feature vector pair with the highest similarity or multiple feature vector pairs with high similarities.

Next, the feature vector pair that meets the preset condition is filtered out as the abnormal feature vectors. The similarity between two feature vectors may be calculated by using a Euclidean distance algorithm or another similarity algorithm, which is not limited in the embodiments in this application. For example, for each of all feature vectors outputted by the feature generator and the feature extractor, another feature vector that is most similar to the feature vector is determined, and labels of the two feature vectors are compared to determine whether the labels are the same. If the labels of the two feature vectors are different, for example, if the label of one feature vector is 1, and the label of the other is 0, the two feature vectors are determined as a feature vector pair that meets a preset condition, and the two feature vectors are filtered out as abnormal feature vectors.

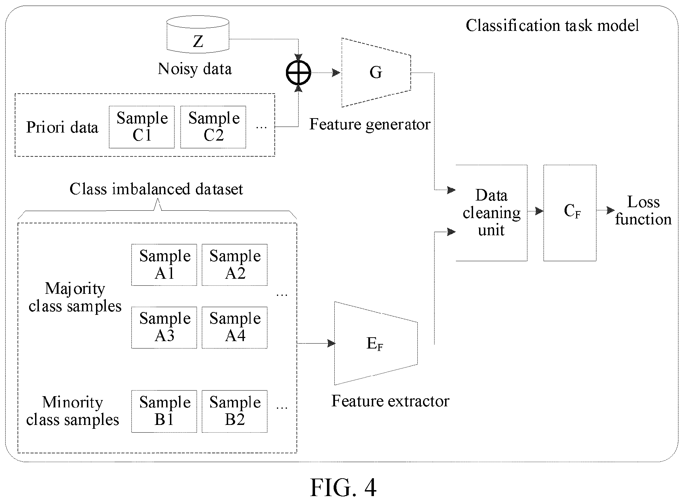

FIG. 4 exemplarily shows a schematic structural diagram of a classification task model. The classification task model includes a feature generator G, a feature extractor E_{F}, a classifier C_{F}, and a data cleaning unit. An output of the feature generator G and an output of the feature extractor E_{F} are connected to an input of the data cleaning unit, and an output of the data cleaning unit is connected to an input of the classifier C_{F}. The feature extractor E_{F} has the same structure and configuration as the feature extractor E_{I} in the classification task model shown in FIG. 2, and the classifier C_{F} has the same structure and configuration as the classifier Cr in the classification task model shown in FIG. 2. The first dataset (including majority class samples and minority class samples) is used to train the classification task model. When a preset training stop condition is met, the training of the classification task model is stopped, and the classification task model is obtained. The preset training stop condition may be that, for example, the accuracy of the model reaches a preset requirement, the number of times for training reaches a preset number, or a time length of training reaches a preset time length, which is not limited in the embodiments of this application.

In summary, according to the technical solution provided in the embodiments of this application, a feature generator is obtained by training a generative adversarial network, and minority class samples (that is, a class of training samples of a smaller number in a class imbalanced dataset) are augmented in a feature space by using the feature generator. Augmentation is performed at a feature aspect instead of simply duplicating minority class samples by up-sampling, so that overfitting in a finally trained classification task model is prevented, thus improving the accuracy of the final trained classification task model.

In addition, according to the technical solution provided in the embodiments of this application, during the training process of the classification task model, abnormal feature vectors outputted by the feature generator and the feature extractor are filtered out by using the data cleaning unit, so that abnormal feature vectors generated by the feature generator are suppressed, thereby further improving the accuracy of the finally trained classification task model.

In addition, in the embodiments of this application, the input of the feature generator does not completely include noisy data. If the feature vector similar to a real sample is generated completely based on the noisy data, there is a lack of effective constraint. Therefore, the input of the feature generator includes a superposition of a priori data and noisy data, which can prevent problems of non-convergence and potential failure during the training process of the generative adversarial network, thus increasing the robustness of the generative adversarial network.

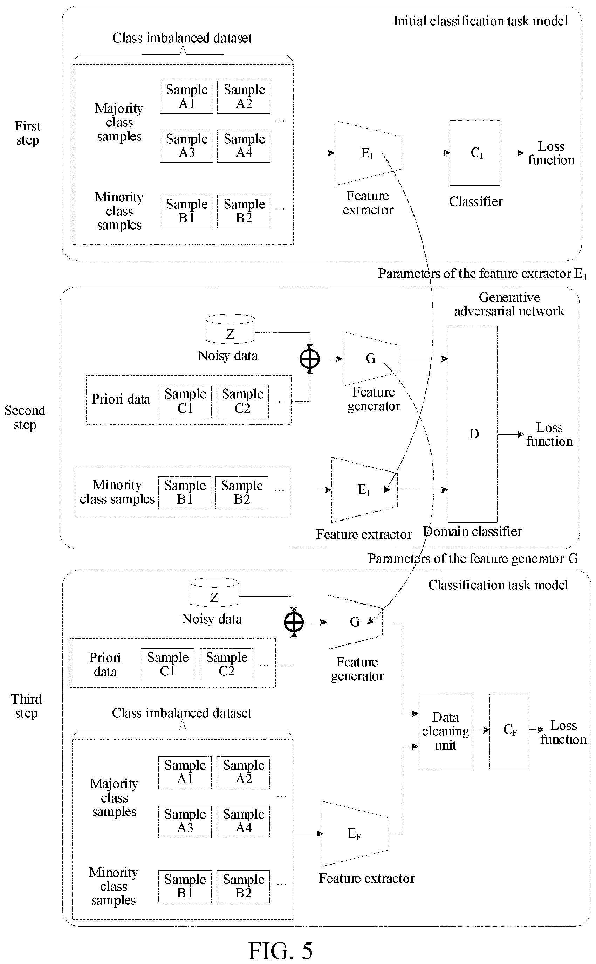

Hereinafter, the technical solution provided in the embodiments of this application is described with reference to FIG. 5. The training process of the classification task model provided in the embodiments of this application may include the following three steps.

In a first step, an initial feature extractor is trained.

In this step, an initial classification task model is constructed, which includes a feature extractor E_{I} and a classifier C_{I}, and the initial classification task model is trained by using a class imbalanced dataset to obtain a feature extractor E_{I}.

In a second step, a feature generator is trained.

In this step, a generative adversarial network is constructed, which includes the initially trained feature extractor E_{I}, an initial feature generator G, and a domain classifier D. During the training process, parameters of the feature extractor E_{I} are fixed, and the feature generator G is obtained by training the generative adversarial network.

In a third step, a final classification task model is trained.

In this step, a classification task model is constructed, which includes a feature generator G, a feature extractor E_{F}, a data cleaning unit, and a classifier E_{F}. During the training process, parameters of the feature generator G are fixed, for an original class imbalanced dataset, the feature generator G is used to augment minority class samples in the feature space, so that a class imbalanced learning task is transformed to a class balanced learning task, and a finally trained classification task model is obtained.

The technical solution provided in the embodiments of this application is applicable to a model training process of a machine learning classification task in the AI field, and is particularly suitable for a training process of a classification task model with a training dataset being a class imbalanced dataset. Taking a classification task of a class imbalanced medical image, a training dataset includes multiple sub-images extracted from medical images, where some of the sub-images are positive samples (that is, images of lesion regions), and some are negative samples (that is, images of non-lesion regions), and the number of the negative samples is much larger than the number of the positive samples. In this application scenario, the classification task model may be referred to as a radiology lesion determining model. An input of the model includes a sub-image extracted from a medical image, and an output includes a determining result of whether the sub-image represents a lesion region. A feature generator is obtained by training a generative adversarial network, the feature generator is used to augment minority class sample in a feature space, and finally a more accurate radiology lesion determining model is trained to assist a doctor in diagnosis and analysis of lesions, for example, detection and analysis of lumps in a mammography image.

This solution is performed for test by using a dataset including 2194 mammography images and a Camelyon 2016 pathology image dataset. Regions of interest (ROI) are extracted from the images to obtain a set of sub-images, and imbalance ratios of 1:10 and 1:20 are used. Test results are shown in Table-1 and Table-2 below.

**Table-1**

| Class imbalanced ratio | Evaluation indicator | Solution 1 | Solution 2 | Solution 3 | Solution 4 | Solution 5 | Solution 6 |
|---|---|---|---|---|---|---|---|
| 1:10 | Acc | 0.8379 | 0.8555 | 0.8287 | 0.8423 | 0.8430 | 0.8665 |
| | AUC | 0.8452 | 0.8878 | 0.8204 | 0.9071 | 0.9082 | 0.9083 |
| 1:20 | Acc | 0.8087 | 0.8180 | 0.8047 | 0.8125 | 0.8070 | 0.8258 |
| | AUC | 0.7797 | 0.8417 | 0.7798 | 0.8453 | 0.8587 | 0.8704 |

**Table-2**

| Class imbalanced ratio | Evaluation indicator | Solution 1 | Solution 2 | Solution 3 | Solution 4 | Solution 5 | Solution 6 |
|---|---|---|---|---|---|---|---|
| 1:10 | Acc | 0.8999 | 0.9243 | 0.8882 | 0.9062 | 0.9023 | 0.9307 |
| | AUC | 0.9157 | 0.9417 | 0.9047 | 0.9597 | 0.9601 | 0.9615 |
| 1:20 | Acc | 0.8516 | 0.8887 | 0.8506 | 0.8760 | 0.8809 | 0.9043 |
| | AUC | 0.8921 | 0.9175 | 0.8764 | 0.9521 | 0.9424 | 0.9459 |

Table-1 shows a test result based on a mammography image dataset, and Table-2 shows a test result based on a Camelyon 2016 pathology image dataset.

In Table-1 and Table-2, Solution 1 represents that no processing is performed on the datasets. Solution 2 represents that the datasets are down-sampled. Solution 3 represents that the datasets are up-sampled. Solution 4 represents that the datasets are augmented in a sample space. Solution 5 represents that the datasets are augmented in a feature space by using the technical solution of this application and a data cleaning step is not included. Solution 6 represents that the datasets are augmented in a feature space by using the technical solution of this application and a data cleaning step is included.

In Table-1 and Table-2, Acc (accuracy) and AUC (area under the ROC curve) are both model evaluation parameters. Acc represents an accuracy of a finally trained classification task model. A large Acc represents that the model has a good performance, and a small Acc represents that the model has a poor performance. AUC represents an area under a receiver operating characteristic (ROC) curve. AUC intuitively reflects a classification ability expressed by the ROC curve. A large AUC represents that the model has a good performance, and a small AUC represents that the model has a poor performance.

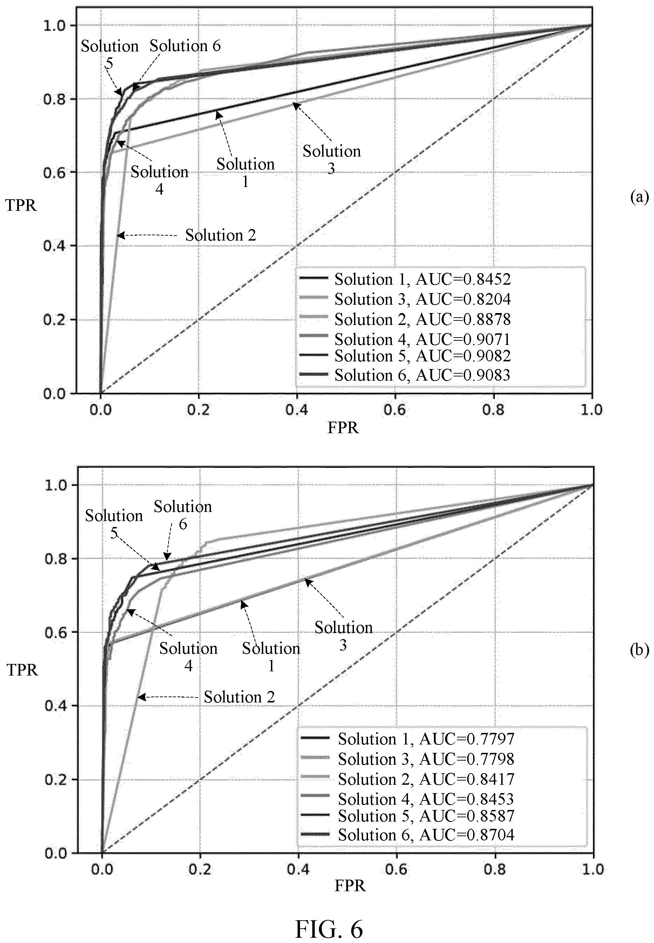

The part (a) in FIG. 6 shows ROC curves and corresponding AUC values of the six solutions when using the mammography image datasets, with the class imbalance ratio of 1: 10. The part (b) in FIG. 6 shows ROC curves and corresponding AUC values of the six solutions when using the mammography image datasets, with the class imbalance ratio of 1:20.

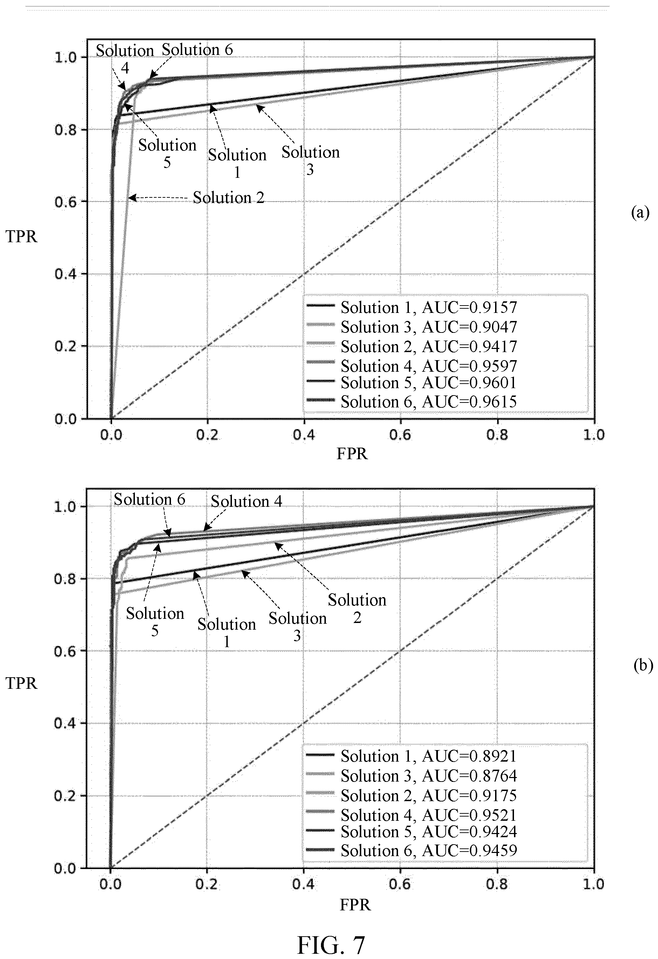

The part (a) in FIG. 7 shows ROC curves and corresponding AUC values of the six solutions when using the Camelyon 2016 pathology image datasets, with the class imbalance ratio of 1:10. The part (b) in FIG. 7 shows ROC curves and corresponding AUC values of the six solutions when using the Camelyon 2016 pathology image datasets, with the class imbalance ratio of 1:20.

It can be seen from graphs of the test results that the technical solution of this application has a better effect than other solutions such as up-sampling, down-sampling, and sample space augmentation technologies. In addition, if the data cleaning step is added, the solution can further improve the performance of the finally trained classification task model.

The following describes apparatus embodiments of this application, which can be used for executing the method embodiments of this application. For details not disclosed in the apparatus embodiments of this application, refer to the method embodiments of this application.

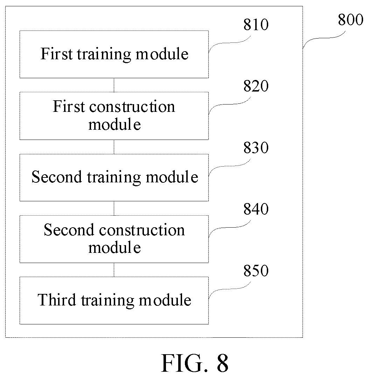

FIG. 8 is a block diagram of an apparatus for training a classification task model according to an embodiment of this application. The apparatus has functions of implementing the foregoing method examples. The functions may be implemented by using hardware, or may be implemented by executing corresponding software by the hardware. The apparatus may be a computer device or may be arranged in a computer device. The apparatus 800 may include: a first training module 810, a first construction module 820, a second training module 830, a second construction module 840, and a third training module 850.

The first training module 810 is configured to train an initial feature extractor by using a first dataset to obtain a feature extractor. The first dataset is a class imbalanced dataset including first class samples and second class samples, the number of the first class samples is greater than the number of the second class samples, and the first dataset is determined based on medical images.

The first construction module 820 is configured to construct a generative adversarial network. The generative adversarial network includes the feature extractor and an initial feature generator, and the initial feature generator is configured to generate a feature vector of the same dimension as the feature extractor.

The second training module 830 is configured to train the generative adversarial network by using the second class samples to obtain a feature generator.

The second construction module 840 is configured to construct a classification task model, the classification task model includes the feature generator and the feature extractor.

The third training module 850 is configured to train the classification task model by using the first dataset, the feature generator is configured to augment the second class samples in a feature space in a training process.

In summary, according to the technical solution provided in the embodiments of this application, a feature generator is obtained by training a generative adversarial network, and minority class samples (that is, a class of training samples of the smaller number in a class imbalanced dataset) are augmented in a feature space by using the feature generator. Augmentation is performed at a feature aspect instead of simply duplicating minority class samples by up-sampling, so that overfitting in a finally trained classification task model is prevented, thus improving the accuracy of the final trained classification task model.

In some possible designs, the generative adversarial network further includes a domain classifier. The domain classifier is configured to distinguish between a feature vector outputted by the feature extractor and a feature vector outputted by the feature generator. The second training module 830 is further configured to perform a first parameter update and a second parameter update in each training process of the generative adversarial network. The first parameter update includes: assigning a first label to an input of the feature extractor, and assigning a second label to an input of the feature generator; calculating a first loss function value of the domain classifier; and updating parameters of the domain classifier based on the first loss function value. The second parameter update includes: blocking the input of the feature extractor, and assigning the first label to the input of the feature generator; calculating a second loss function value of the domain classifier; and updating parameters of the feature generator based on the second loss function value.

In some possible designs, an input of the initial feature generator includes a superposition of a priori data and noisy data. The a priori data is extracted from the second class samples of the first dataset, or the a priori data is extracted from samples in a second dataset that are of the same class as the second class samples.

In some possible designs, the classification task model further includes a data cleaning unit. The third training module is further configured to: filter out, by using the data cleaning unit, abnormal feature vectors outputted by the feature generator and the feature extractor.

In some possible designs, the third training module is further configured to: select, by using the data cleaning unit, a feature vector pair that meets a preset condition from the feature vectors outputted by the feature generator and the feature extractor, the feature vector pair that meets the preset condition including two feature vectors with different labels and a similarity that is greater than a threshold; and filter out the feature vector pair that meets the preset condition as the abnormal feature vectors.

In some possible designs, the first training module 810 is further configured to: construct an initial classification task model, the initial classification task model including the initial feature extractor and an initial classifier; and train the initial classification task model by using the first dataset to obtain the feature extractor.

It is to be noted that when the apparatus provided in the foregoing embodiments implements functions of the apparatus, the division of the foregoing functional modules is merely used as an example for description. In practice, the functions may be assigned to and completed by different functional modules according to requirements, that is, the internal structure of the device is divided into different functional modules, to implement all or some of the functions described above. In addition, the apparatus and method embodiments provided in the foregoing embodiments belong to the same concept. For the specific implementation process, reference may be made to the method embodiments, and details are not described herein again.

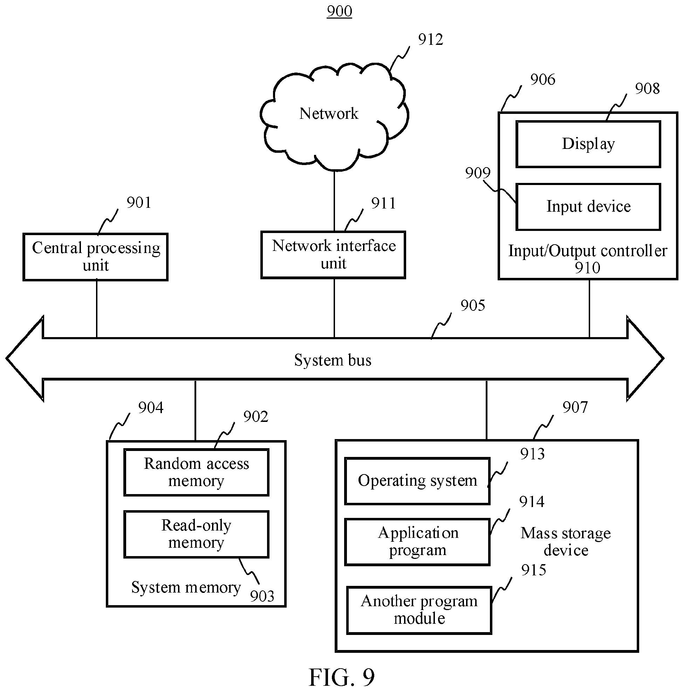

FIG. 9 is a schematic structural diagram of a computer device according to an embodiment of this application. The computer device may be any electronic device with a data processing function and a data storage function, for example, a PC or a server. The computer device is configured to perform the method for training a classification task model in the foregoing embodiments.

The computer device 900 includes a central processing unit (CPU) 901, a system memory 904 including a random access memory (RAM) 902 and a read-only memory (ROM) 903, and a system bus 905 connecting the system memory 904 and the CPU 901. The computer device 900 further includes a basic input/output (I/O) system 906 configured to transmit information between components in a computer, and a mass storage device 907 configured to store an operating system 913, an application 914, and another program module 915.

The basic I/O system 906 includes a display 908 configured to display information, and an input device 909 used by a user to input information, such as a mouse or a keyboard. The display 908 and the input device 909 are both connected to the CPU 901 by using an I/O controller 910 connected to the system bus 905. The basic I/O system 906 may further include the I/O controller 910 for receiving and processing input from multiple other devices such as a keyboard, a mouse, an electronic stylus, or the like. Similarly, the I/O controller 910 further provides output to a display screen, a printer, or another type of output device.

The mass storage device 907 is connected to the CPU 901 through a mass storage controller (not shown) connected to the system bus 905. The mass storage device 907 and an associated computer-readable medium provide non-volatile storage for the computer device 900. In other words, the mass storage device 907 may include a computer-readable medium (not shown) such as a hard disk or a CD-ROM drive.

Without loss of generality, the computer-readable media may include a computer storage medium and a communication medium. The computer storage medium includes volatile and non-volatile medium, and removable and non-removable medium implemented by using any method or technology used for storing information such as computer-readable instructions, data structures, program modules, or other data. The computer-storage medium includes a RAM, a ROM, an EPROM, an EEPROM, a flash memory or another solid-state storage technology, a CD-ROM, a DVD or another optical storage, a magnetic cassette, a magnetic tape, or a magnetic disk storage or another magnetic storage device. In addition, a person skilled in the art may know that the computer storage medium is not limited to the foregoing several types. The system memory 904 and the mass storage device 907 may be collectively referred to as a memory.

According to the various embodiments of this application, the computer device 900 may further be connected, through a network such as the Internet, to a remote computer on the network for running. That is, the computer device 900 may be connected to a network 912 by using a network interface unit 911 connected to the system bus 905, or may be connected to another type of network or a remote computer system (not shown) by using a network interface unit 911.

The memory stores at least one instruction, at least one section of program, a code set, or an instruction set, and the at least one instruction, the at least one section of program, the code set, or the instruction set is configured to be executed by one or more processors to implement the method for training a classification task model provided in the foregoing embodiments.

In an exemplary embodiment, a computer-readable storage medium is further provided in the embodiments of this application. The storage medium is configured to store a computer program, the computer program is configured to perform the method for training a classification task model provided in the foregoing embodiments. In an exemplary embodiment, the computer-readable storage medium may be a ROM, a RAM, a CD-ROM, a magnetic tape, a floppy disk, an optical data storage device.

In an exemplary embodiment, a computer program product is further provided. The computer program product, when executed, performs the method for training a classification task model provided in the foregoing embodiments.

It is to be noted that "multiple" mentioned herein means two or more. "And/or" is used to describe an association relationship for describing associated objects and represents that three relationships may exist. For example, A and/or B may represent the following three cases: only A exists, both A and B exist, and only B exists. The character "/" generally indicates an "or" relationship between the associated objects. In addition, the numbers of steps described herein merely exemplarily show a possible execution sequence of the steps. In some other embodiments, the steps may not be performed according to the number sequence. For example, two steps with different numbers may be performed simultaneously, or two steps with different numbers may be performed according to a sequence contrary to the sequence shown in the figure. This is not limited in the embodiments of this application.

The foregoing descriptions are merely examples of the embodiments of this application, but are not intended to limit this application.

## Claims

1. A method for training a classification task model based on medical images, the method being performed by a computer device, the method comprising:
training (101) an initial feature extractor by using a first dataset to obtain a feature extractor, the first dataset being a class imbalanced dataset which comprises first class samples and second class samples, the first class samples being negative samples, and the second class samples being positive samples, the number of the first class samples being greater than the number of the second class samples, the first dataset being determined based on medical images, the first dataset comprising a plurality of sub-images of non-lesion regions and lesion regions that are extracted from the medical images ;
constructing (102) a generative adversarial network, the generative adversarial network comprising the feature extractor and an initial feature generator, the initial feature generator being configured to generate a feature vector of the same dimension as the feature extractor;
training (103) the generative adversarial network by using the second class samples to obtain a feature generator, the feature generator being used to augment the second class samples in a feature space during a training process;
constructing (104) a classification task model, the classification task model comprising the feature generator and the feature extractor; and
training (105) the classification task model by using the first dataset, the trained classification task model being used to perform lesion classification based on a medical image.

2. The method according to claim 1, wherein the generative adversarial network further comprises a domain classifier, the domain classifier is configured to distinguish between a feature vector outputted by the feature extractor and a feature vector outputted by the feature generator, and the training the generative adversarial network by using the second class samples to obtain a feature generator comprises:
performing a first parameter update and a second parameter update during each training process of the generative adversarial network, wherein
the first parameter update comprises:
assigning a first label to an input of the feature extractor, and assigning a second label to an input of the initial feature generator;
calculating a first loss function value of the domain classifier; and
updating parameters of the domain classifier based on the first loss function value,
the second parameter update comprises:
blocking the input of the feature extractor, and assigning the first label to the input of the initial feature generator;
calculating a second loss function value of the domain classifier; and
updating parameters of the initial feature generator based on the second loss function value.

3. The method according to claim 1, wherein an input of the initial feature generator comprises a superposition of priori data and noisy data, and
the priori data is extracted from the second class samples of the first dataset, or the priori data is extracted from samples in a second dataset that are of the same class as the second class samples, the second dataset is another dataset different from the first dataset in the same task.

4. The method according to any one of claims 1 to 3, wherein the classification task model further comprises a data cleaning unit, and in a process of training the classification task model by using the first dataset, the method further comprises:
filtering out, by using the data cleaning unit, abnormal feature vectors outputted by the feature generator and the feature extractor.

5. The method according to claim 4, wherein the filtering out, by using the data cleaning unit, abnormal feature vectors outputted by the feature generator and the feature extractor comprises:
selecting, by using the data cleaning unit, a feature vector pair that meets a preset condition from feature vectors outputted by the feature generator and the feature extractor, the feature vector pair that meets the preset condition comprising two feature vectors with different labels and a similarity that is greater than a threshold; and
filtering out the feature vector pair that meets the preset condition as the abnormal feature vectors.

6. The method according to any one of claims 1 to 3, the training an initial feature extractor by using a first dataset to obtain a feature extractor comprises:
constructing an initial classification task model, the initial classification task model comprising the initial feature extractor; and
training the initial classification task model by using the first dataset to obtain the feature extractor.

7. The method according to any one of claims 1 to 6, wherein during the training the generative adversarial network, parameters of the feature extractor are fixed.

8. An apparatus (800) for training a classification task model based on medical images, **characterized in that** the apparatus comprising:
a first training module (810), configured to train an initial feature extractor by using a first dataset to obtain a feature extractor, the first dataset being a class imbalanced dataset which comprises first class samples and second class samples, the first class samples being negative samples, and the second class samples being positive samples, the number of the first class samples being greater than the number of the second class samples, the first dataset being determined based on medical images, the first dataset comprises a plurality of sub-images of non-lesion regions and lesion regions that are extracted from the medical images ;
a first construction module (820), configured to construct a generative adversarial network, the generative adversarial network comprising the feature extractor and an initial feature generator, the initial feature generator being configured to generate a feature vector of the same dimension as the feature extractor;
a second training module (830), configured to train the generative adversarial network by using the second class samples to obtain a feature generator, the feature generator being used to augment the second class samples in a feature space during a training process;
a second construction module (840), configured to construct a classification task model, the classification task model comprising the trained feature generator and feature extractor; and
a third training module (850), configured to train the classification task model by using the first dataset, the trained classification task model being used to perform lesion classification based on a medical image.

9. The apparatus (800) according to claim 8, wherein the generative adversarial network further comprises a domain classifier, the domain classifier is configured to distinguish between a feature vector outputted by the feature extractor and a feature vector outputted by the feature generator, and the second training module (830) is further configured to:
perform a first parameter update and a second parameter update during each training process of the generative adversarial network, wherein
the first parameter update comprises:
assign a first label to an input of the feature extractor, and assign a second label to an input of the feature generator;
calculate a first loss function value of the domain classifier; and
update parameters of the domain classifier based on the first loss function value,
the second parameter update comprises:
blocking the input of the feature extractor, and assigning the first label to the input of the feature generator;
calculating a second loss function value of the domain classifier; and
updating parameters of the feature generator based on the second loss function value.

10. The apparatus (800) according to claim 8, wherein an input of the initial feature generator comprises a superposition of a priori data and noisy data, and
the priori data is extracted from the second class samples of the first dataset, or the a priori data is extracted from samples in a second dataset that are of the same class as the second class samples, the second dataset is another dataset different from the first dataset in the same task.

11. The apparatus (800) according to any one of claims 8 to 10, wherein the classification task model further comprises a data cleaning unit, and the third training module (850) is further configured to:
filter out, by using the data cleaning unit, abnormal feature vectors outputted by the feature generator and the feature extractor.

12. The apparatus (800) according to claim 11, wherein the third training module (850) is further configured to:
select, by using the data cleaning unit, a feature vector pair that meets a preset condition from the feature vectors outputted by the feature generator and the feature extractor, the feature vector pair that meets the preset condition comprising two feature vectors with different labels and a similarity that is greater than a threshold; and
filter out the feature vector pair that meets the preset condition as the abnormal feature vectors.

13. The apparatus (800) according to any one of claims 8 to 10, wherein the first training module (810) is further configured to:
construct an initial classification task model, the initial classification task model comprising the initial feature extractor; and
train the initial classification task model by using the first dataset to obtain the feature extractor.

14. A computer-readable storage medium, configured to store a computer program, the computer program being configured to perform the method according to any one of claims 1 to 7.

15. A computer program product comprising instructions, the instructions, when run on a computer, causing the computer to perform the method for training a classification task model according to any one of claims 1 to 7.

## Patentansprüche

1. Verfahren zum Trainieren eines Klassifizierungsaufgabenmodells basierend auf medizinischen Bildern, wobei das Verfahren von einer Computervorrichtung durchgeführt wird, wobei das Verfahren umfasst:
Trainieren (101) eines anfänglichen Merkmalsextraktors durch Verwenden eines ersten Datensatzes, um einen Merkmalsextraktor zu erhalten, wobei der erste Datensatz ein klassenunausgeglichener Datensatz ist, der erste Klassenproben und zweite Klassenproben umfasst, wobei die ersten Klassenproben Negativproben sind und die zweiten Klassenproben Positivproben sind, wobei die Anzahl der ersten Klassenproben größer ist als die Anzahl der zweiten Klassenproben, der erste Datensatz basierend auf medizinischen Bildern bestimmt wird und der erste Datensatz mehrere Unterbilder von nicht Nicht-Läsionsregionen und Läsionsregionen umfasst, die aus den medizinischen Bildern extrahiert sind;
Konstruieren (102) eines generativen adversariellen Netzes, wobei das generative adversarielle Netz den Merkmalsextraktor und einen anfänglichen Merkmalsgenerator umfasst, wobei der anfängliche Merkmalsgenerator dafür ausgelegt ist, einen Merkmalsvektor derselben Dimension zu generieren wie der Merkmalsextraktor;
Trainieren (103) des generativen adversariellen Netzes durch Verwenden der zweiten Klassenproben, um einen Merkmalsgenerator zu erhalten, wobei der Merkmalsgenerator verwendet wird, um die zweiten Klassenproben während eines Trainingsprozesses in einem Merkmalsraum zu augmentieren;
Konstruieren (104) eines Klassifizierungsaufgabenmodells, wobei das Klassifizierungsaufgabenmodell den Merkmalsgenerator und den Merkmalsextraktor umfasst; und
Trainieren (105) des Klassifizierungsaufgabenmodells durch Verwenden des ersten Datensatzes, wobei das trainierte Klassifizierungsaufgabenmodell verwendet wird, um eine Läsionsklassifizierung basierend auf einem medizinischen Bild durchzuführen.

2. Verfahren gemäß Anspruch 1, wobei das generative adversarielle Netz ferner einen Domänenklassifizierer umfasst, wobei der Domänenklassifizierer dafür ausgelegt ist, zwischen einem Merkmalsvektor, der von dem Merkmalsextraktor ausgegeben wird, und einem Merkmalsvektor, der von dem Merkmalsgenerator ausgegeben wird, zu unterscheiden, und das Trainieren des generativen adversariellen Netzes durch Verwenden der zweiten Klassenproben zum Erhalten eines Merkmalsgenerators umfasst:
Durchführen einer ersten Parameteraktualisierung und einer zweiten Parameteraktualisierung während jedes Trainingsprozesses des generativen adversariellen Netzes,
wobei die erste Parameteraktualisierung umfasst:
Zuweisen einer ersten Beschriftung zu einer Eingabe des Merkmalsextraktors und Zuweisen einer zweiten Beschriftung zu einer Eingabe des anfänglichen Merkmalsgenerators;
Berechnen eines ersten Verlustfunktionswertes des Domänenklassifizierers; und
Aktualisieren von Parametern des Domänenklassifizierers basierend auf dem ersten Verlustfunktionswert,
wobei die zweite Parameteraktualisierung umfasst:
Blockieren der Eingabe des Merkmalsextraktors und Zuweisen der ersten Beschriftung zu der Eingabe des anfänglichen Merkmalsgenerators;
Berechnen eines zweiten Verlustfunktionswertes des Domänenklassifizierers; und
Aktualisieren von Parametern des anfänglichen Merkmalsgenerators basierend auf dem zweiten Verlustfunktionswert.

3. Verfahren gemäß Anspruch 1, wobei eine Eingabe des anfänglichen Merkmalsgenerators eine Überlagerung von A-priori-Daten und verrauschten Daten umfasst, und
die A-priori-Daten aus den zweiten Klassenproben des ersten Datensatzes extrahiert sind oder die A-priori-Daten aus Stichproben in einem zweiten Datensatz extrahiert sind, die von derselben Klasse stammen wie die zweiten Klassenproben, wobei der zweite Datensatz ein anderer Datensatz ist, der von dem ersten Datensatz in derselben Aufgabe verschieden ist.

4. Verfahren gemäß einem der Ansprüche 1 bis 3, wobei das Klassifizierungsaufgabenmodell ferner eine Datenbereinigungseinheit umfasst und wobei, in einem Prozess des Trainierens des Klassifizierungsaufgabenmodells durch Verwenden des ersten Datensatzes, das Verfahren ferner umfasst:
Herausfiltern, durch Verwenden der Datenbereinigungseinheit, anormaler Merkmalsvektoren, die von dem Merkmalsgenerator und dem Merkmalsextraktor ausgegeben werden.

5. Verfahren gemäß Anspruch 4, wobei das Herausfiltern, durch Verwenden der Datenbereinigungseinheit, anormaler Merkmalsvektoren, die von dem Merkmalsgenerator und dem Merkmalsextraktor ausgegeben werden, umfasst:
Auswählen, durch Verwenden der Datenbereinigungseinheit, eines Merkmalsvektorpaares, das eine voreingestellte Bedingung erfüllt, aus Merkmalsvektoren, die von dem Merkmalsgenerator und dem Merkmalsextraktor ausgegeben werden, wobei das Merkmalsvektorpaar, das die voreingestellte Bedingung erfüllt, zwei Merkmalsvektoren mit unterschiedlichen Beschriftungen und einer Ähnlichkeit umfasst, die größer als ein Schwellwert ist; und
Herausfiltern des Merkmalsvektorpaares, das die voreingestellte Bedingung erfüllt, als die anormalen Merkmalsvektoren.

6. Verfahren gemäß einem der Ansprüche 1 bis 3, wobei das Trainieren eines anfänglichen Merkmalsextraktors durch Verwenden eines ersten Datensatzes zum Erhalten eines Merkmalsextraktors umfasst:
Konstruieren eines anfänglichen Klassifizierungsaufgabenmodells, wobei das anfängliche Klassifizierungsaufgabenmodell den anfänglichen Merkmalsextraktor umfasst; und
Trainieren des anfänglichen Klassifizierungsaufgabenmodells durch Verwenden des ersten Datensatzes, um den Merkmalsextraktor zu erhalten.

7. Verfahren gemäß einem der Ansprüche 1 bis 6, wobei während des Trainings des generativen adversariellen Netzes Parameter des Merkmalsextraktors fest vorgegeben sind.

8. Einrichtung (800) zum Trainieren eines Klassifizierungsaufgabenmodells basierend auf medizinischen Bildern, **dadurch gekennzeichnet, dass** die Einrichtung umfasst:
ein erstes Trainingsmodul (810), das dafür ausgelegt ist, einen anfänglichen Merkmalsextraktor durch Verwenden eines ersten Datensatzes zu trainieren, um einen Merkmalsextraktor zu erhalten, wobei der erste Datensatz ein klassenunausgeglichener Datensatz ist, der erste Klassenproben und zweite Klassenproben umfasst, wobei die ersten Klassenproben Negativproben sind und die zweiten Klassenproben Positivproben sind, wobei die Anzahl der ersten Klassenproben größer ist als die Anzahl der zweiten Klassenproben, der erste Datensatz basierend auf medizinischen Bildern bestimmt wird und der erste Datensatz mehrere Unterbilder von nicht Nicht-Läsionsregionen und Läsionsregionen umfasst, die aus den medizinischen Bildern extrahiert sind;
ein erstes Konstruktionsmodul (820), das dafür ausgelegt ist, ein generatives adversarielles Netz zu konstruieren, wobei das generative adversarielle Netz den Merkmalsextraktor und einen anfänglichen Merkmalsgenerator umfasst, wobei der anfängliche Merkmalsgenerator dafür ausgelegt ist, einen Merkmalsvektor derselben Dimension zu generieren wie der Merkmalsextraktor;
ein zweites Trainingsmodul (830), das dafür ausgelegt ist das generative adversarielle Netz durch Verwenden der zweiten Klassenproben zu trainieren, um einen Merkmalsgenerator zu erhalten, wobei der Merkmalsgenerator verwendet wird, um die zweiten Klassenproben während eines Trainingsprozesses in einem Merkmalsraum zu augmentieren;
ein zweites Konstruktionsmodul (840), das dafür ausgelegt ist, ein Klassifizierungsaufgabenmodell zu konstruieren, wobei das Klassifizierungsaufgabenmodell den trainierten Merkmalsgenerator und den Merkmalsextraktor umfasst; und
ein drittes Trainingsmodul (850), das dafür ausgelegt ist, das Klassifizierungsaufgabenmodell durch Verwenden des ersten Datensatzes zu trainieren, wobei das trainierte Klassifizierungsaufgabenmodell verwendet wird, um eine Läsionsklassifizierung basierend auf einem medizinischen Bild durchzuführen.

9. Einrichtung (800) gemäß Anspruch 8, wobei das generative adversarielle Netz ferner einen Domänenklassifizierer umfasst, wobei der Domänenklassifizierer dafür ausgelegt ist, zwischen einem Merkmalsvektor, der von dem Merkmalsextraktor ausgegeben wird, und einem Merkmalsvektor, der von dem Merkmalsgenerator ausgegeben wird, zu unterscheiden, und das zweite Trainingsmodul (830) ferner ausgelegt ist zum:
Durchführen einer ersten Parameteraktualisierung und einer zweiten Parameteraktualisierung während jedes Trainingsprozesses des generativen adversariellen Netzes, wobei
die erste Parameteraktualisierung umfasst:
Zuweisen einer ersten Beschriftung zu einer Eingabe des Merkmalsextraktors und Zuweisen einer zweiten Beschriftung zu einer Eingabe des Merkmalsgenerators;
Berechnen eines ersten Verlustfunktionswertes des Domänenklassifizierers; und
Aktualisieren von Parametern des Domänenklassifizierers basierend auf dem ersten Verlustfunktionswert,
wobei die zweite Parameteraktualisierung umfasst:
Blockieren der Eingabe des Merkmalsextraktors und Zuweisen der ersten Beschriftung zu der Eingabe des Merkmalsgenerators;
Berechnen eines zweiten Verlustfunktionswertes des Domänenklassifizierers; und
Aktualisieren von Parametern des Merkmalsgenerators basierend auf dem zweiten Verlustfunktionswert.

10. Einrichtung (800) gemäß Anspruch 8, wobei eine Eingabe des anfänglichen Merkmalsgenerators eine Überlagerung von A-priori-Daten und verrauschten Daten umfasst, und
die A-priori-Daten aus den zweiten Klassenproben des ersten Datensatzes extrahiert sind oder die A-priori-Daten aus Stichproben in einem zweiten Datensatz extrahiert sind, die von derselben Klasse stammen wie die zweiten Klassenproben, wobei der zweite Datensatz ein anderer Datensatz ist, der von dem ersten Datensatz in derselben Aufgabe verschieden ist.

11. Einrichtung (800) gemäß einem der Ansprüche 8 bis 10, wobei das Klassifizierungsaufgabenmodell ferner eine Datenbereinigungseinheit umfasst und das dritte Trainingsmodul (850) ferner ausgelegt ist zum:
Herausfiltern, durch Verwenden der Datenbereinigungseinheit, anormaler Merkmalsvektoren, die von dem Merkmalsgenerator und dem Merkmalsextraktor ausgegeben werden.

12. Einrichtung (800) gemäß Anspruch 11, wobei das dritte Trainingsmodul (850) ferner ausgelegt ist zum:
Auswählen, durch Verwenden der Datenbereinigungseinheit, eines Merkmalsvektorpaares, das eine voreingestellte Bedingung erfüllt, aus den Merkmalsvektoren, die von dem Merkmalsgenerator und dem Merkmalsextraktor ausgegeben werden, wobei das Merkmalsvektorpaar, das die voreingestellte Bedingung erfüllt, zwei Merkmalsvektoren mit unterschiedlichen Beschriftungen und einer Ähnlichkeit umfasst, die größer als ein Schwellwert ist; und
Herausfiltern des Merkmalsvektorpaares, das die voreingestellte Bedingung erfüllt, als die anormalen Merkmalsvektoren.

13. Einrichtung (800) gemäß einem der Ansprüche 8 bis 10, wobei das erste Trainingsmodul (810) ferner ausgelegt ist zum:
Konstruieren eines anfänglichen Klassifizierungsaufgabenmodells, wobei das anfängliche Klassifizierungsaufgabenmodell den anfänglichen Merkmalsextraktor umfasst; und
Trainieren des anfänglichen Klassifizierungsaufgabenmodells durch Verwenden des ersten Datensatzes, um den Merkmalsextraktor zu erhalten.

14. Computerlesbares Datenspeichermedium, das dafür ausgelegt ist, ein Computerprogramm zu speichern, wobei das Computerprogramm dafür ausgelegt ist, das Verfahren gemäß einem der Ansprüche 1 bis 7 durchzuführen.

15. Computerprogrammprodukt, umfassend Anweisungen, wobei die Anweisungen, wenn sie auf einem Computer ausgeführt werden, den Computer veranlassen, das Verfahren zum Trainieren eines Klassifizierungsaufgabenmodells gemäß einem der Ansprüche 1 bis 7 durchzuführen.

## Revendications

1. Procédé d'entraînement d'un modèle de tâches de classification sur la base d'images médicales, le procédé étant réalisé par un dispositif informatique, le procédé comprenant :
l'entraînement (101) d'un extracteur de caractéristiques initial à l'aide d'un premier jeu de données afin d'obtenir un extracteur de caractéristiques, le premier jeu de données étant un jeu de données à classes déséquilibrées comprenant des premiers échantillons de classe et des deuxièmes échantillons de classe, les premiers échantillons de classe étant des échantillons négatifs et les deuxièmes échantillons de classe étant des échantillons positifs, le nombre des premiers échantillons de classe étant supérieur au nombre des deuxièmes échantillons de classe, le premier jeu de données étant déterminé sur la base d'images médicales, le premier jeu de données comprenant une pluralité de sous-images de régions non lésionnelles et de régions lésionnelles qui sont extraites des images médicales ;
la construction (102) d'un réseau antagoniste génératif, le réseau antagoniste génératif comprenant l'extracteur de caractéristiques et un générateur de caractéristiques initial, le générateur de caractéristiques initial étant configuré pour générer un vecteur de caractéristiques de la même dimension que l'extracteur de caractéristiques ;
l'entraînement (103) du réseau antagoniste génératif à l'aide des deuxièmes échantillons de classe afin d'obtenir un générateur de caractéristiques, le générateur de caractéristiques étant utilisé pour augmenter les deuxièmes échantillons de classe dans un espace de caractéristiques au cours d'un processus d'entraînement ;
la construction (104) d'un modèle de tâches de classification, le modèle de tâches de classification comprenant le générateur de caractéristiques et l'extracteur de caractéristiques ; et
l'entraînement (105) du modèle de tâches de classification à l'aide du premier jeu de données, le modèle de tâches de classification étant utilisé pour réaliser une classification lésionnelle sur la base d'une image médicale.

2. Procédé selon la revendication 1, le réseau antagoniste génératif comprenant en outre un classificateur de domaine, le classificateur de domaine étant configuré pour établir une distinction entre un vecteur de caractéristiques délivré par l'extracteur de caractéristiques et un vecteur de caractéristiques délivré par le générateur de caractéristiques, et l'entraînement du réseau antagoniste génératif à l'aide des deuxièmes échantillons de classe afin d'obtenir un générateur de caractéristiques comprenant :
la réalisation d'une première mise à jour de paramètres et d'une deuxième mise à jour de paramètres au cours de chaque processus d'entraînement du réseau antagoniste génératif,
la première mise à jour de paramètres comprenant :
l'assignation d'une première étiquette à une entrée de l'extracteur de caractéristiques, et l'assignation d'une deuxième étiquette à une entrée du générateur de caractéristiques initial ;
le calcul d'une première valeur de fonction de perte du classificateur de domaine ; et
la mise à jour de paramètres du classificateur de domaine sur la base de la première valeur de fonction de perte,
la deuxième mise à jour de paramètres comprenant :
le blocage de l'entrée de l'extracteur de caractéristiques, et l'assignation de la première étiquette à l'entrée du générateur de caractéristiques initial ;
le calcul d'une deuxième valeur de fonction de perte du classificateur de domaine ; et
la mise à jour de paramètres du générateur de caractéristiques initial sur la base de la deuxième valeur de fonction de perte.

3. Procédé selon la revendication 1, une entrée du générateur de caractéristiques initial comprenant une superposition de données a priori et de données bruitées, et
les données a priori étant extraites des deuxièmes échantillons de classe du premier jeu de données, ou les données a priori étant extraites d'échantillons dans un deuxième jeu de données qui sont de la même classe que les deuxièmes échantillons de classe, le deuxième jeu de données étant un autre jeu de données différent du premier jeu de données dans la même tâche.

4. Procédé selon l'une quelconque des revendications 1 à 3, le modèle de tâches de classification comprenant en outre une unité de nettoyage de données et, dans un processus d'entraînement du modèle de tâches de classification à l'aide du premier jeu de données, le procédé comprenant en outre :
l'élimination par filtrage, à l'aide de l'unité de nettoyage de données, de vecteurs de caractéristiques anormaux délivrés par le générateur de caractéristiques et l'extracteur de caractéristiques.

5. Procédé selon la revendication 4, l'élimination par filtrage, à l'aide de l'unité de nettoyage de données, de vecteurs de caractéristiques anormaux délivrés par le générateur de caractéristiques et l'extracteur de caractéristiques comprenant :
la sélection, à l'aide de l'unité de nettoyage de données, d'une paire de vecteurs de caractéristiques qui satisfait à une condition prédéfinie parmi des vecteurs de caractéristiques délivrés par le générateur de caractéristiques et l'extracteur de caractéristiques, la paire de vecteurs de caractéristiques qui satisfait à la condition prédéfinie comprenant deux vecteurs de caractéristiques dont les étiquettes sont différentes et qui présentent une similarité supérieure à un seuil ; et
l'élimination par filtrage, en tant que vecteurs de caractéristiques anormaux, de la paire de vecteurs de caractéristiques qui satisfait à la condition prédéfinie.

6. Procédé selon l'une quelconque des revendications 1 à 3, l'entraînement d'un extracteur de caractéristiques initial à l'aide d'un premier jeu de données afin d'obtenir un extracteur de caractéristiques comprenant :
la construction d'un modèle de tâches de classification initial, le modèle de tâches de classification initial comprenant l'extracteur de caractéristiques initial ; et
l'entraînement du modèle de tâches de classification initial à l'aide du premier jeu de données afin d'obtenir l'extracteur de caractéristiques.

7. Procédé selon l'une quelconque des revendications 1 à 6, au cours de l'entraînement du réseau antagoniste génératif, les paramètres de l'extracteur de caractéristiques étant fixés.

8. Appareil (800) d'entraînement d'un modèle de tâches de classification sur la base d'images médicales, l'appareil étant **caractérisé en ce qu'**il comprend :
un premier module d'entraînement (810), configuré pour entraîner un extracteur de caractéristiques initial à l'aide d'un premier jeu de données afin d' obtenir un extracteur de caractéristiques, le premier jeu de données étant un jeu de données à classes déséquilibrées comprenant des premiers échantillons de classe et des deuxièmes échantillons de classe, les premiers échantillons de classe étant des échantillons négatifs et les deuxièmes échantillons de classe étant des échantillons positifs, le nombre des premiers échantillons de classe étant supérieur au nombre des deuxièmes échantillons de classe, le premier jeu de données étant déterminé sur la base d'images médicales, le premier jeu de données comprenant une pluralité de sous-images de régions non lésionnelles et de régions lésionnelles qui sont extraites des images médicales ;
un premier module de construction (820), configuré pour construire un réseau antagoniste génératif, le réseau antagoniste génératif comprenant l'extracteur de caractéristiques et un générateur de caractéristiques initial, le générateur de caractéristiques initial étant configuré pour générer un vecteur de caractéristiques de la même dimension que l'extracteur de caractéristiques ;
un deuxième module d'entraînement (830), configuré pour entraîner le réseau antagoniste génératif à l'aide des deuxièmes échantillons de classe afin d'obtenir un générateur de caractéristiques, le générateur de caractéristiques étant utilisé pour augmenter les deuxièmes échantillons de classe dans un espace de caractéristiques au cours d'un processus d'entraînement ;
un deuxième module de construction (840), configuré pour construire un modèle de tâches de classification, le modèle de tâches de classification comprenant le générateur de caractéristiques et l'extracteur de caractéristiques entraînés ; et
un troisième module d'entraînement (850), configuré pour entraîner le modèle de tâches de classification à l'aide du premier jeu de données, le modèle de tâches de classification étant utilisé pour réaliser une classification lésionnelle sur la base d'une image médicale.

9. Appareil (800) selon la revendication 8, le réseau antagoniste génératif comprenant en outre un classificateur de domaine, le classificateur de domaine étant configuré pour établir une distinction entre un vecteur de caractéristiques délivré par l'extracteur de caractéristiques et un vecteur de caractéristiques délivré par le générateur de caractéristiques, et le deuxième module d'entraînement (830) étant configuré en outre pour :
réaliser une première mise à jour de paramètres et une deuxième mise à jour de paramètres au cours de chaque processus d'entraînement du réseau antagoniste génératif,
la première mise à jour de paramètres comprenant :
l'assignation d'une première étiquette à une entrée de l'extracteur de caractéristiques, et l'assignation d'une deuxième étiquette à une entrée du générateur de caractéristiques ;
le calcul d'une première valeur de fonction de perte du classificateur de domaine ; et
la mise à jour de paramètres du classificateur de domaine sur la base de la première valeur de fonction de perte,
la deuxième mise à jour de paramètres comprenant :
le blocage de l'entrée de l'extracteur de caractéristiques, et l'assignation de la première étiquette à l'entrée du générateur de caractéristiques ;
le calcul d'une deuxième valeur de fonction de perte du classificateur de domaine ; et
la mise à jour de paramètres du générateur de caractéristiques sur la base de la deuxième valeur de fonction de perte.

10. Appareil (800) selon la revendication 8, une entrée du générateur de caractéristiques initial comprenant une superposition de données a priori et de données bruitées, et
les données a priori étant extraites des deuxièmes échantillons de classe du premier jeu de données, ou les données a priori étant extraites d'échantillons dans un deuxième jeu de données qui sont de la même classe que les deuxièmes échantillons de classe, le deuxième jeu de données étant un autre jeu de données différent du premier jeu de données dans la même tâche.

11. Appareil (800) selon l'une quelconque des revendications 8 à 10, le modèle de tâches de classification comprenant en outre une unité de nettoyage de données, et le troisième module d'entraînement (850) étant configuré en outre pour :
éliminer par filtrage, à l'aide de l'unité de nettoyage de données, des vecteurs de caractéristiques anormaux délivrés par le générateur de caractéristiques et l'extracteur de caractéristiques.

12. Appareil (800) selon la revendication 11, le troisième module d'entraînement (850) étant configuré en outre pour :
sélectionner, à l'aide de l'unité de nettoyage de données, une paire de vecteurs de caractéristiques qui satisfait à une condition prédéfinie parmi les vecteurs de caractéristiques délivrés par le générateur de caractéristiques et l'extracteur de caractéristiques, la paire de vecteurs de caractéristiques qui satisfait à la condition prédéfinie comprenant deux vecteurs de caractéristiques dont les étiquettes sont différentes et qui présentent une similarité supérieure à un seuil ; et
éliminer par filtrage, en tant que vecteurs de caractéristiques anormaux, la paire de vecteurs de caractéristiques qui satisfait à la condition prédéfinie.

13. Appareil (800) selon l'une quelconque des revendications 8 à 10, le premier module d'entraînement (810) étant configuré en outre pour :
construire un modèle de tâches de classification initial, le modèle de tâches de classification initial comprenant l'extracteur de caractéristiques initial ; et
entraîner le modèle de tâches de classification initial à l'aide du premier jeu de données afin d'obtenir l'extracteur de caractéristiques.

14. Support de stockage lisible par ordinateur, configuré pour stocker un programme d'ordinateur, le programme d'ordinateur étant configuré pour réaliser le procédé selon l'une quelconque des revendications 1 à 7.

15. Produit-programme d'ordinateur comprenant des instructions, les instructions, lorsqu'elles s'exécutent sur un ordinateur, amenant l'ordinateur à réaliser le procédé d'entraînement d'un modèle de tâches de classification selon l'une quelconque des revendications 1 à 7.
